# EUROPÄISCHE PATENTSCHRIFT

(11) **EP 3 648 658 B1**
(45) Veröffentlichungstag und Bekanntmachung des Hinweises auf die Patenterteilung: **17.03.2021**
(21) Anmeldenummer: 19752661.9
(22) Anmeldetag: 30.07.2019
(51) Int. Cl.: A61B 3/08, A61B 3/00, A61B 3/103, A61B 3/15, A61B 3/032

(54) **BINOKULARES EINBLICKGERÄT ZUM PRÜFEN DER AUGEN EINER PERSON UND ZUGEHÖRIGES VERFAHREN**
BINOCULAR SIGHT TESTING DEVICE FOR TESTING THE EYES OF A PERSON, AND ASSOCIATED METHOD
TESTEUR DE VUE BINOCULAIRE POUR EXAMINER LES YEUX D'UNE PERSONNE ET PROCÉDÉ CORRESPONDANT

(30) Priorität: 25.09.2018 DE 102018123605
(43) Veröffentlichungstag der Anmeldung: 13.05.2020
(73) Patentinhaber: ONE GmbH & Co. KG, 75417 Mühlacker (DE)
(72) Erfinder: STELZER, Lars-Erik, 75233 Tiefenbronn (DE)
(74) Vertreter: DREISS Patentanwälte PartG mbB
(86) Internationale Anmeldenummer: PCT/EP2019/070538
(87) Internationale Veröffentlichungsnummer: WO 2020/064178

(56) Entgegenhaltungen:
- DE-A1- 19 901 963
- US-A1- 2005 280 777
- US-A1- 2014 362 345

## Beschreibung

Die Erfindung betrifft ein binokulares Einblickgerät zum Prüfen der Augen einer Person, mit einem Gehäuse, mit zwei Einblicköffnungen zum Einblicken in das Gehäuse entlang jeweils einer Einblickachse, wobei im Gehäuse für jedes Auge eine Messeinheit mit einem Bildschirm zur Erzeugung von Sehzeichen vorgesehen ist.

Ein derartiges binokulares Einblickgerät wird beispielsweise unter der Bezeichnung "Oculus Binoptometer" von der Firma Oculus Optikgeräte GmbH, 35582 Wetzlar, vertrieben. Bei einem derartigen Gerät blickt die Person durch die Einblicköffnungen in das Gerät, wobei ein im Gerät vorgesehener Bildschirm Sehzeichen darbietet. Das Einblickgerät dient insbesondere als Sehtestgerät, mit dem beispielsweise eine Farbsinnprüfung, ein Binokulartest, ein NunKontrastsehtest oder auch Dämmerungssehen und eine Blendempfindlichkeit durchgeführt werden kann. Zur Prüfung einer Übersichtigkeit oder einer Kurzsichtigkeit können Korrektionsgläser vor das Einblickgerät vorgeschalten werden.

Derartige Einblickgeräte haben den Vorteil, dass sie vergleichsweise kompakt bauen, portabel sind und dass störende Lichtquellen die Augenmessung nicht beeinflussen.

Ferner sind aus dem Stand der Technik Autorefraktometer bekannt, welche zur Bestimmung der objektiven Refraktion vorgesehen sind. Diese Geräte basieren auf der Infrarotprojektion von Lichtfiguren auf dem Augenhintergrund, deren messbares Bild mit Hilfe von vorgeschalteten Linsen scharf gestellt wird oder die in Koinzidenz gebracht werden. Bei der bekannten Autorefraktionsmessung blickt die Person in der Regel auf ein Bild, das einen fernen Abstand von mehreren Metern bis zur Unendlichkeit zur Person aufweist.

Die Kombination eines objektiven Autorefraktometers mit einem Wellenfrontanalysesystem und einem Phoroptersystem, welches eine verstellbare Linsenanordnung umfasst, ist beispielsweise aus der WO 2016/178237 A1 bekannt.

Aus der WO 2013/150513 A1 ist eine andere Kombination einer Phoroptermessung und einer Wellenfrontanalyse zur Durchführung einer objektiven Phoroptermessung in mehreren Iterationsschritten bekannt.

Aus der US 2005/280777A1 ist ein binokularer Apparat in mit Merkmalen des Oberbegriffs des Patentanspruchs eins bekannt. Aus der DE 199 01 963 A1 ist ein Stereomikroskop und der US 2014/362345A1 ein binokulares ophthalmologisches Gerät bekannt.

Der vorliegenden Erfindung liegt die Aufgabe zugrunde, ein binokulares Einblickgerät bereitzustellen, mit dem insbesondere eine objektive Bestimmung der Refraktion auf einfache und dennoch funktionssichere Art und Weise durchgeführt werden kann.

Diese Aufgabe wird gelöst durch ein binokulares Einblickgerät mit den Merkmalen des Patentanspruchs 1.

Gemäß der Erfindung ist folglich vorgesehen, dass die Messeinheit für jedes Auge eine verstellbare Sehschärfenlinsenanordnung zur Bestimmung der Refraktion und insbesondere des Nah- und/oder Fernpunkts des jeweiligen Auges umfasst, dass im Gehäuse für jedes Auge eine entlang einer X-Achse und/oder einer Y-Achse verfahrbare Ausrichteinheit zum Ausrichten des vom jeweiligen Bildschirm erzeugten Lichtpfades bzw. der Einblickachsen des Geräts hin in das jeweilige Auge vorgesehen ist, und dass ferner das Einblickgerät so eingerichtet ist, dass eine durch ein Verfahren der jeweiligen Ausrichteinheit sich ergebende Distanzänderung des Lichtpfads zwischen dem Bildschirm und dem Augenhintergrund ausgeglichen wird. Bei der X-Achse kann es sich insbesondere um eine Horizontalachse handeln. Die Y-Achse verläuft vorzugsweise senkrecht zur X-Achse und senkrecht zur Einblickachse.

Dabei ist zu berücksichtigen, dass die Länge des Lichtpfads zwischen dem Bildschirm und dem Augenhintergrund maßgeblichen Einfluss auf die Sehschärfenmessung und damit auf die Bestimmung der Refraktion bzw. des Nah- und/oder Fernpunkts des jeweiligen Auges hat. Eine Veränderung der Distanz des Lichtpfads zwischen dem Bildschirm und dem Augenhintergrund führt folglich zu einer Verfälschung des Messergebnisses. Erfindungsgemäß wird eine auftretende Distanzänderung ausgeglichen, so dass bei der Messung der Sehschärfe keine Ungenauigkeiten aufgrund einer Längenänderung des Lichtpfades auftreten können.

Ferner ist vorgesehen, dass eine Ausgleichseinheit zum Ausgleichen der Distanzänderung des Lichtpfads zwischen dem Bildschirm und dem Augenhintergrund vorgesehen ist. Die Ausgleichseinheit kann den Ausgleich beispielsweise auf mechanische Art und Weise so bewirken, dass die Messeinheit so verfahren wird, dass eine auftretende Distanzänderung aufgrund des Verfahrens der jeweiligen Ausrichteinheit durch entsprechendes, gegenläufiges Verfahren der jeweiligen Messeinheit ausgeglichen bzw. kompensiert wird. Ein Ausgleich kann beispielsweise durch eine mechanische Kopplung der einzelnen verfahrbaren Bauteilen, insbesondere zwischen der Ausrichteinheit und der Messeinheit so bereitgestellt werden, dass die Distanz zwischen dem Augenhintergrund und dem Bildschirm stets konstant ist. Andererseits ist denkbar, dass ein geregeltes System zur Verfügung gestellt wird, und zwar derart, ein Verfahrwert der jeweiligen Ausrichteinheit bestimmt wird und dass die jeweilige Messeinheit in Abhängigkeit von dem bestimmten Verfahrwert der jeweiligen Ausrichteinheit mittels der Ausgleichseinheit verfahren wird, so dass letztlich die Distanz zwischen dem Augenhintergrund und dem jeweiligen Bildschirm stets konstant bleibt.

Eine weitere Art des Ausgleichs einer Distanzänderung bei der Sehschärfenmessung kann dadurch erfolgen, dass bei der Berechnung der Fehlsichtigkeit die Distanzänderung berücksichtigt wird. Dieses Verfahren beruht folglich auf einer Korrekturberechnung der Fehlsichtigkeit.

Eine weitere Möglichkeit eines Ausgleichs einer sich ergebenden Distanzänderung besteht darin, dass eine zusätzliche Linsenausgleichsanordnung vorgesehen werden kann, die die aufgetretene Distanzänderung im Strahlengang kompensiert.

Das binokulare Einblickgerät hat ferner den Vorteil, dass die Sehschärfe der Person und insbesondere eine objektive Refraktion mit einem in sich abgeschlossenen Gerät, dem Einblickgerät, bestimmt werden kann. Da beide Augen in das Einblickgerät blicken, kann die Sehschärfe für das eine und/oder andere Auge bestimmt werden, wobei das jeweils andere Auge durch Wahrnehmung eines geeignetes Sehzeichens in einem natürlichen, und dadurch entspannten Zustand gehalten werden kann. Insgesamt wird dadurch die Qualität der Sehschärfenmessung erheblich gesteigert. Da die Sehzeichen im Einblickgerät generiert werden, kann auch kein störendes Fremdlicht auftreten, welches eine Messung beeinflussen kann. Ferner ist es nicht erforderlich, die Sehzeichen in einem Abstand von mehreren Metern zur Person darzubieten. Die Sehzeichen werden innerhalb des Gerätes auf dem jeweiligen Bildschirm in der Ferne oder im Unendlichen dargeboten. Insgesamt kann mit dem erfindungsgemäßen Einblickgerät eine Sehschärfenmessung auf kleinstem Raum mit höchster Präzision erfolgen.

Dabei ist denkbar, dass eine Messeinrichtung vorgesehen ist, die die Differenz von einer Referenzlage hin zu einer jeweiligen Ist-Lage der jeweiligen Ausrichteinheit misst. Die Messeinrichtung kann dabei vorteilhafterweise innerhalb der Messeinheit vorgesehen sein und so angeordnet und ausgebildet sein, dass mit ihr die Distanz zwischen Bildschirm und Augenhintergrund bestimmt werden kann. Auf Basis der von der Messeinheit bestimmten Messwerte kann folglich der Ausgleich vorgenommen werden. Die Messeinheit kann beispielsweise eine auf die IR Licht basierende Lasermesseinrichtung sein. Die gemessene Differenz wird dann bei der Bestimmung der Sehschärfe des jeweiligen Auges berücksichtigt und ausgeglichen. Mit der Messeinrichtung kann folglich unmittelbar der Abstand zwischen dem Bildschirm und dem Augenhintergrund bestimmt werden. Allerdings ist auch denkbar, dass der Abstand mittelbar bestimmt wird, beispielsweise durch Messen der Verfahrbewegung der jeweiligen Ausrichteinheit und Bestimmen des sich daraus ergebenden Abstands zwischen dem Augenhintergrund und dem Bildschirm.

Gemäß einer Weiterbildung der Erfindung ist vorgesehen, dass die jeweilige Messeinheit nicht nur den Bildschirm, der beispielsweise als Mikro-Farbdisplay ausgebildet sein kann, umfasst, sondern auch eine IR-Lichtquelle zur Erzeugung von definierten Lichtfiguren auf dem Augenhintergrund, einen IR-Sensor zur Detektion von an dem Augenhintergrund reflektierten Lichtfiguren, und einen Linsenantrieb zur Verstellung der Sehschärfenlinsenanordnung. Ferner ist eine Steuereinrichtung derart vorgesehen und eingerichtet, dass sie in Abhängigkeit von den mit den jeweiligen IR-Sensor detektierten Lichtfiguren die jeweilige Sehschärfenlinsenanordnung zur Bestimmung der Refraktion bzw. des Nah- und/oder Fernpunkts des jeweiligen Auges verstellt. Die Steuereinrichtung steuert dann den Linsenantrieb in Abhängigkeit von den detektierten Lichtfiguren an. Bei der IR-Lichtquelle kann es sich beispielsweise um einen Infrarot-Laser handeln oder auch um eine Infrarotlicht aussendende Leuchtdiode. Bei dem IR-Sensor kann es sich beispielsweise um einen Hartmann-Shack-Sensor handeln. Mit der Sehschärfenlinsenanordnung wird insbesondere die Sehschärfe bei maximaler Fernakkommodation bestimmt.

Die IR-Lichtquelle kann beispielsweise zudem auch als Messeinrichtung zur Messung des Abstands zwischen dem jeweiligen Bildschirm hin zum jeweiligen Augenhintergrund Verwendung finden.

Gemäß der Erfindung kann ferner vorgesehen sein, dass in der Messeinheit eine HH-Kamera zur Erfassung von der Hornhaut reflektiertem Licht vorgesehen ist, und dass wenigstens ein Ausrichtantrieb zum Verfahren der jeweiligen Ausrichteinheit vorgesehen ist,
wobei die Steuereinrichtung weiter derart eingerichtet ist, dass sie in Abhängigkeit von dem von den HH-Sensoren der HH-Kamera erfassten Lichts den Ausrichtantrieb derart ansteuert, dass das vom jeweiligen Bildschirm erzeugte Licht entlang der Einblickachse in die Augenmitte des jeweiligen Auges fällt. Mit der HH-Kamera kann letztlich die jeweilige Augenmitte bestimmt werden. Dadurch wird gewährleistet, dass das Bildschirmlicht entlang der innerhalb des Gehäuses vorgesehenen Einblickachse aus dem Gehäuse mittig in das jeweilige Auge eintritt. Über die Ausrichteinheiten wird folglich eine Selbstzentrierung der Einblickachsen so erreicht, dass die Person entlang den Einblickachsen in das Einblickgerät blickt. Die Einblickachsen richten sich dabei insbesondere entlang den Pupillenachsen aus, sofern die Person ohne Winkelfehler in das Gerät blickt. Die Distanz der beiden Einblickachsen entspricht dann der Pupillendistanz der Person. Insgesamt wird hierdurch der Vorteil erzielt, dass die Person lediglich in die Einblicköffnungen blicken muss; über die Ausrichteinheiten erfolgt eine Selbstausrichtung des Systems hin zur jeweiligen Augenmitte der Person.

Dabei ist denkbar, dass die jeweilige Ausrichteinheit eine erste Umlenkeinheit und eine zweite Umlenkeinheit umfasst, wobei die erste Umlenkeinheit das Bildschirmlicht nicht hin zur zweiten Umlenkeinheit umleitet und die zweite Umlenkeinheit das von der ersten Umlenkeinheit kommende Bildschirmlicht hin zur Eintrittsöffnung und letztlich in das jeweilige Auge umleitet. Die zweite Umlenkeinheit ist dabei vorzugsweise entlang einer quer zur Einblickachse verlaufenden X-Achse, die insbesondere die Horizontalachse sein kann, verfahrbar angeordnet, wobei die erste Umlenkeinheit entlang einer senkrecht zur X-Achse und senkrecht zur Einblickachse verlaufenden Y-Achse, die beispielsweise die Vertikalachse sein kann, verfahrbar angeordnet ist. Durch Vorsehen der ersten und der zweiten Umlenkeinheit kann folglich ein Ausrichten der Ausrichteinheit entlang der X-Achse und entlang der Y-Achse unabhängig voneinander erfolgen, bis die jeweilige Einblickachse in der Augenmitte der Person liegt.

Dabei ist denkbar, dass die zweite Umlenkeinheit an der ersten Umlenkeinheit entlang der X-Achse verfahrbar angeordnet ist. Dies ist insbesondere dann vorteilhaft, wenn der Lichtpfad zwischen den beiden Umlenkeinheiten entlang der X-Achse verlaufend angeordnet ist, und wenn der Lichtpfad zwischen der ersten Umlenkeinheit und der Messeinheit entlang der Y-Achse verlaufend angeordnet ist. Ein Verfahren der ersten Umlenkeinheit entlang der Y-Achse und ein Verfahren der zweiten Umlenkeinheit entlang der X-Achse kann dann parallel zu den jeweiligen Lichtpfaden erfolgen.

Ferner ist vorteilhaft, wenn die zweite Umlenkeinheit an der ersten Umlenkeinheit entlang der X-Achse verfahrbar angeordnet ist. Die zweite Umlenkeinheit wird dann zusammen mit der ersten Umlenkeinheit entlang der Y-Achse verfahren. Dies ist insbesondere dann vorteilhaft, wenn der Lichtpfad zwischen der zweiten Umlenkeinheit und der ersten Umlenkeinheit parallel zur X-Achse verläuft und wenn der Lichtpfad zwischen der ersten Umlenkeinheit und der Messeinheit parallel zur Y-Achse verläuft.

Die erste Umlenkeinheit ist dabei vorzugsweise an der Messeinheit entlang ebenfalls der Y-Achse verfahrbar angeordnet.

Ferner ist vorteilhaft, wenn die jeweilige Messeinheit entlang der Y-Achse unabhängig von der jeweiligen Ausrichteinheit bzw. deren Umlenkeinheit in Y-Richtung verfahrbar angeordnet ist. Mit einem Verfahren der zweiten Umlenkeinheit entlang der X-Achse und der ersten Umlenkeinheit, zusammen mit der zweiten Umlenkeinheit, entlang der Y-Achse kann folglich die zweite Umlenkeinheit auf die Augenmitte eingestellt werden. Ergibt sich dadurch eine Distanzänderung eines Lichtpfads zwischen dem Bildschirm und dem Augenhintergrund kann diese Distanzänderung insbesondere durch entsprechendes Verfahren der Messeinheit in Y-Richtung, und zwar unabhängig von einem Verfahren der Ausrichteinheiten in Y-Richtung, ausgeglichen werden.

Bei Auftreten einer Distanzänderung kann ein Verfahren der Messeinheit insbesondere über eine geeignete mechanische Kopplung der Messeinheit mit der ersten Umlenkeinheit und/oder der zweiten Umlenkeinheit erfolgen. Andererseits ist auch denkbar, dass die Steuereinrichtung derart eingerichtet ist, dass sie die erste Umlenkeinheit, die zweite Umlenkeinheit und/oder die Messeinheit, bzw. deren Antriebe, derart ansteuert, dass die Länge des Lichtpfads zwischen dem Augenhintergrund und dem jeweiligen Bildschirm stets konstant ist.

Gemäß der Erfindung ist ferner vorteilhaft, wenn die erste Umlenkeinheit einen Strahlvereiniger aufweist, der das vom Bildschirm kommende Bildschirmlicht und das von der IR-Lichtquelle kommende IR-Licht in einem zum jeweiligen Auge führenden Lichtpfad vereint.

Dabei ist denkbar, dass der Strahlvereiniger zugleich als Strahlteiler ausgebildet ist, der das an dem Augenhintergrund reflektierte IR-Licht und das an der Hornhaut reflektierte Licht in einen zur HH-Kamera führenden Lichtpfad und einem zum HS-Sensor führenden Lichtpfad teilt. Insgesamt kann dadurch der Strahlvereiniger mehrere Aufgaben übernehmen, wodurch das Einblickgerät einen vergleichsweise kleinen Aufbau aufweist.

Ferner ist denkbar, dass in der Messeinheit ein Strahlenumlenker vorgesehen ist, der das an der Hornhaut reflektierte Licht hin zur HH-Kamera umlenkt.

Ferner ist vorteilhaft, wenn im Lichtpfad des Lichts der IR-Lichtquelle ein Strahlenumlenker vorgesehen ist, der das am Augenhintergrund reflektierte IR-Licht aus dem Strahlenpfad hin zum HS-Sensor umlenkt.

Eine weitere vorteilhafte Ausgestaltung der Erfindung ergibt sich dann, wenn die jeweilige Ausrichteinheit, und bei Vorsehen von den beiden Umlenkeinheiten, die jeweils zweite Umlenkeinheit, einen hinter der jeweiligen Einblicköffnung liegenden Prismenkompensator zur Korrektur eines Winkelfehlers zwischen der Pupillenachse und der Einblickachse (Phorie) und/oder der Korrektur einer Konvergenz aufweist. Dadurch, dass die prismatischen Wirkungen addierbar sind, können beide Funktionen von einem Prismenkompensator erfüllt werden. Insgesamt wird dadurch gewährleistet, dass eine Phorie und/oder Konvergenz bei der Bestimmung von Refraktionswerten in der Nähe ausgeglichen wird.

Gemäß der Erfindung ist es weiter vorteilhaft, wenn die Sehschärfenlinsenanordnung so ausgebildet ist, dass unterschiedliche Linsen für unterschiedliche Messungen in den Strahlenpfad eingebracht werden können. Insbesondere können damit auch unterschiedliche Messmethoden durchgeführt werden. Denkbar ist, dass beispielsweise ein Kreuzzylinder für die Messung einer Stabsichtigkeit/eines Astigmatismus sowie dessen Lage bestimmt und auch ausgeglichen werden kann.

Ferner ist vorteilhaft, wenn die Steuereinheit derart eingerichtet ist, dass sie die beiden Bildschirme derart ansteuert, dass die Person dreidimensionale Bilder und/oder bewegte Bilder wahrnimmt. Durch Wahrnehmen von dreidimensionalen Bildern und/oder von bewegten Bildern können realitätsnahe Bedingungen während der Augenmessung erreicht werden, wodurch letztlich das Messergebnis verbessert werden kann.

Eine weitere vorteilhafte Ausgestaltung der Erfindung sieht eine Kommunikationseinheit vor, die derart eingerichtet ist und mit der Steuereinheit derart kommuniziert, dass die Person über die Kommunikationseinheit mit einer dritten, ortsfernen Person kommunizieren kann. Das Kommunizieren kann über Sprache mit Hilfe einer Sprachausgabe und/oder einer Spracherkennung erfolgen. Die Kommunikation kann insbesondere zur Durchführung einer subjektiven Refraktion erfolgen.

Ebenfalls ist denkbar, dass die Kommunikationseinheit derart eingerichtet ist, dass die Person direkt mit der Steuereinheit kommuniziert. Dabei ist vorteilhaft, wenn die Refraktionsabläufe in der Steuereinheit hinterlegt sind. Insgesamt kann hierdurch eine subjektive Refraktion durch Abfragen eines vorgegebenen Fragenkatalogs ohne fachlich ausgebildetes Personal erfolgen.

Der Aufbau des Geräts ist vorzugsweise derart, dass die Lichtpfade innerhalb des Gehäuses für die beiden Augen symmetrisch zu einer Mittelebene verlaufend angeordnet sind. Vorzugsweise sind die optischen Systeme für die beiden Augen spiegelsymmetrisch aufgebaut.

Um ein angenehmes Einblicken in das Einblickgerät zu ermöglichen, ist vorteilhaft, wenn ein Standfuß vorgesehen ist und wenn das Gehäuse gegenüber dem Standfuß um eine Horizontalachse verschwenkbar und/oder entlang einer Vertikalachse verstellbar angeordnet ist.

Die eingangs genannte Aufgabe wird auch gelöst durch ein Verfahren zum Betreiben insbesondere eines erfindungsgemäßen Einblickgeräts mit den Merkmalen des Patentanspruchs 20.

Weitere Einzelheiten und vorteilhafte Ausgestaltungen der Erfindung sind der nachfolgenden Beschreibung zu entnehmen, anhand derer ein Ausführungsbeispiel der Erfindung näher beschrieben und erläutert ist.

### Es zeigen:

- Figur 1: eine Seitenansicht eines erfindungsgemäßen Einblickgeräts;
- Figur 2: die Vorderansicht des Geräts gemäß Figur 1;
- Figur 3: einen Schnitt durch das Gerät gemäß Figur 1 entlang der Linie III; und
- Figur 4: eine schematische, isometrische Darstellung gemäß der Anordnung der Umlenkeinheiten des Einblickgeräts gemäß Figur 3.

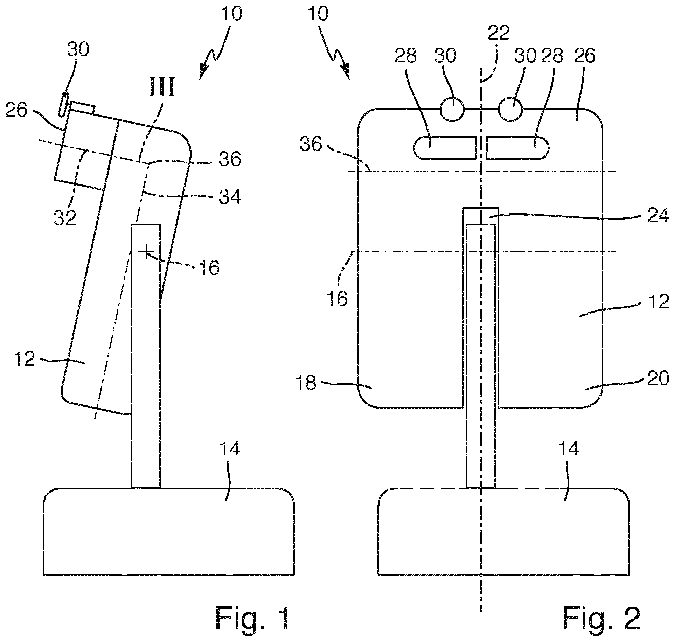

Die Figuren 1 und 2 zeigen ein erfindungsgemäßes binokulares Einblickgerät 10, das ein Gehäuse 12 und einen Standfuß 14 umfasst. Das Gehäuse 12 ist dabei um eine horizontale Schwenkachse 16 gegenüber dem Standfuß 14 verschwenkbar.

Das Gehäuse 12 umfasst ferner zwei Gehäusehälften 18 und 20, die zur Mittelebene 22 symmetrisch aufgebaut sind. Wie aus Figur 2 deutlich wird, greift der Standfuß 14 in eine zwischen den beiden Gehäusehälften 18, 20 vorgesehene Ausnehmung 24 und ist dort an den Gehäusehälften 18, 20 um die Schwenkachse 16 verschwenkbar angeordnet.

Das Gehäuse 12 weist im oberen Bereich, auf einer einer Person zugewandten Seite 26, für jedes Auge 48 eine Einblicköffnung 28 auf, durch welche die Person, deren Augen 48 geprüft werden sollen, in das Gehäuse 12 einblickt.

Um einen definierten Abstand der Person zum Gehäuse 12, bzw. zur Seite 26, zu gewährleisten, sind am Gehäuse 12 zwei Anlageelemente 30 vorgesehen, welche zur Anlage an die Stirn der Person dienen.

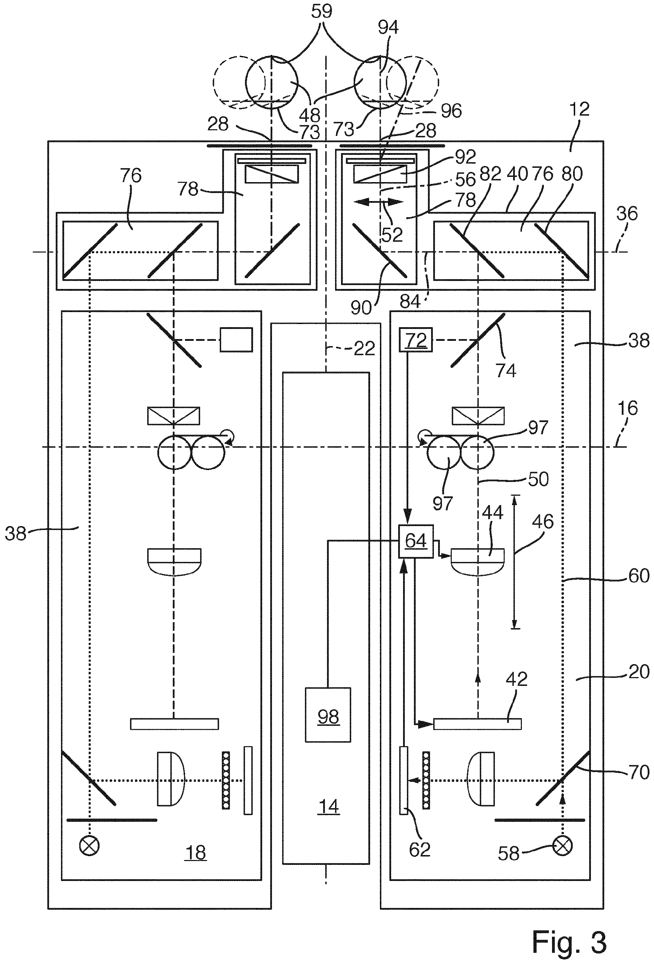

In der Figur 3 sind die beiden Schnittebenen 32 und 34 entlang des Schnittes III aus Figur 1 in einer Ebene dargestellt. Die beiden Schnittebenen 32 und 34 schneiden sich dabei unter einem Winkel von 90° in einer Schnittlinie 36, die in den Figuren 2 und 3 ebenfalls dargestellt ist.

Aus Figur 3 wird deutlich, dass beide Gehäusehälften 18, 20 jeweils eine Messeinheit 38 und eine Ausrichteinheit 40 umfassen. Die jeweilige Messeinheit 38 umfasst jeweils einen Bildschirm 42 in Form eines Mikro-Farbdisplays zur Erzeugung von Sehzeichen sowie eine verstellbare Sehschärfenlinsenanordnung 44, die entlang des Pfeils 46 verstellbar ist. Zur Verstellung ist ein Verstellantrieb vorgesehen, der in der Figur nicht dargestellt ist.

Die jeweilige Sehschärfenlinsenanordnung 44 dient dabei zur Bestimmung der Refraktion sowie des Nah- und/oder Fernpunkts des jeweiligen Auges 48 der Person. Dazu wird in der Messeinheit 38 vom Bildschirm 42 ein Sehzeichen erzeugt, so dass das vom Bildschirm 42 erzeugte Bildschirmlicht entlang des Lichtpfades 50 zunächst die Sehschärfenlinsenanordnung 44 passiert, bevor es die Messeinheit 38 verlässt. In der Ausrichteinheit 40 wird der Lichtpfad 50 hin zum jeweiligen Auge 48 umgelenkt.

Die beiden Ausrichteinheiten 40 sind dabei derart ausgebildet, sie entlang einer X-Achse 52, die vorzugsweise entlang der Horizontalen verläuft, und entlang einer dazu senkrecht verlaufenden Y-Achse 54 verstellbar sind. Die Verstellung erfolgt dabei derart, dass die vorgesehene Einblickachse 56 in das Gehäuse 12 so zum jeweiligen Auge 48 verfahren wird, dass dieses entlang der Einblickachse 56 in das Gehäuse 12 hineinschaut. Die beiden Einblickachsen 56 passen sich folglich jeweils automatisch an die Pupillendistanz der Person an. Durch die Verstellung entlang der Y-Achse 54 kann ferner ein Ausrichten der Einblickachsen 56 an die jeweilige Augenhöhe erfolgen.

Die jeweilige Messeinheit 38 umfasst ferner eine IR-Lichtquelle 58 zur Erzeugung von definierten Lichtfiguren auf dem Augenhintergrund 59. Die erzeugten IR-Lichtfiguren werden entlang des Lichtpfads 60 auf den Augenhintergrund 59 gesendet. Die dort reflektierten Lichtfiguren werden von einem IR-Sensor 62, beispielsweise einem Hartmann-Shack-IR-Sensor, detektiert. Über eine Steuereinrichtung 64 wird die Sehschärfenlinsenanordnung 44 in Abhängigkeit von dem mit dem jeweiligen IR-Sensor 62 detektierten Lichtfiguren zur Bestimmung der Refraktion bzw. des Nah- und/oder Fernpunkts des jeweiligen Auges 48 verstellt. Hierdurch kann eine objektive Autorefraktionsmessung durchgeführt werden.

Zur Umlenkung des am Augenhintergrund 59 reflektierten IR-Lichts ist ein Strahlumlenker 70 vorgesehen, der das am Augenhintergrund 59 reflektierte IR-Licht aus dem Strahlenpfad 60 hin zum IR-Sensor 62 umlenkt.

In der jeweiligen Messeinheit 38 ist ferner eine HH-Kamera 72 vorgesehen, mit der das an der Hornhaut 73 reflektierte Licht, insbesondere das Bildschirmlicht, zur Bestimmung der Augenmitte erfasst wird. Zur Umlenkung des an der Hornhaut 73 reflektierten Lichts ist in der Messeinheit 38 ein Strahlumlenker 74 vorgesehen, der das an der Hornhaut 73 reflektierte Licht hin zur HH-Kamera 72 umlenkt.

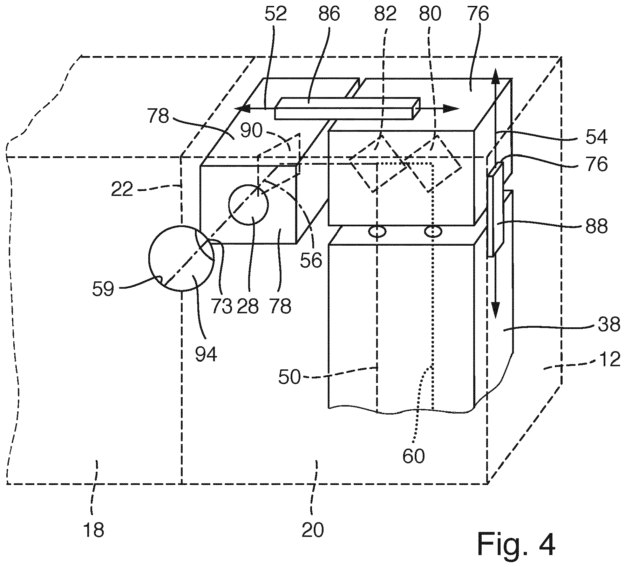

Aus Figur 3 und insbesondere aus der isometrischen Darstellung aus Figur 4 wird deutlich, dass die Ausrichteinheit 40 eine erste Umlenkeinheit 76 und eine zweite Umlenkeinheit 78 umfasst. Die erste Umlenkeinheit 76 dient dabei zur Umlenkung des Bildschirmlichts, bzw. dessen Strahlengang 50, sowie zur Umlenkung des von der IR-Lichtquelle 58 ausgesendeten IR-Lichts bzw. dessen Strahlengang 60 hin zur zweiten Umlenkeinheit 78. Zur Umlenkung des IR-Lichts bzw. des Strahlengangs 60 weist die erste Umlenkeinheit einen Strahlumlenker 80 auf. Zur Einkopplung des vom Bildschirm 42 kommenden Bildschirmlichts in den am Strahlumlenker 80 umgelenkten Strahlengangs 60 in den Strahlengang 50, der vom Bildschirm 42 erzeugt wird, ist in der ersten Umlenkeinheit ein Strahlvereiniger 82 vorgesehen. Der Strahlvereiniger 82 dient für das am Auge reflektierte Licht als Strahlteiler, der das am Augenhintergrund 59 reflektierte IR-Licht und das an der Hornhaut 73 reflektierte Licht in den zur HH-Kamera 72 führenden Lichtpfad und in den zum IR-Sensor 62 führenden Lichtpfad teilt.

Die zweite Umlenkeinheit 78 lenkt den von der ersten Umlenkeinheit 76 kommenden Lichtstrahl 84 hin zur Austrittsöffnung 28 ab. Wie aus der Ansicht gemäß Figur 4 deutlich wird, ist die erste Umlenkeinheit 76 mit der zweiten Umlenkeinheit 78 so bewegungsgekoppelt, dass die zweite Umlenkeinheit 78 entlang einer Führung 86 in Richtung der X-Achse 52 verfahrbar ist. Die erste Umlenkeinheit 76 ist gegenüber der Messeinheit 38 entlang einer Führung 88 entlang der Y-Achse 54 verfahrbar angeordnet. Die X-Achse 52 verläuft dabei im Wesentlichen entlang einer Horizontalachse. Die Y-Achse verläuft dabei vorzugsweise senkrecht zur X-Achse 52 und senkrecht zur Einblickachse 56. Durch Vorsehen von geeigneten, in den Figuren nicht gezeigten Antrieben, die zum einen die zweite Umlenkeinheit 78 entlang der X-Achse 52 und zum anderen die erste Umlenkeinheit 76 zusammen mit der zweiten Umlenkeinheit 78 entlang der Y-Achse 54 verfahren, kann die jeweilige Einblickachse 56 so ausgerichtet werden, dass die Person, bzw. deren Augen 48, entlang der Einblickachse 56 in das Einblickgerät 10 blicken.

Die entsprechenden Antriebe werden dabei von der Steuereinheit 64 in Abhängigkeit von den von der HH-Kamera 72 detektierten, an der Hornhaut 73 reflektierten Bildschirmsignalen so verfahren, dass die jeweilige Einblickachse 56 hin zur jeweiligen Augenmitte ausgerichtet ist. Das Einblickgerät 10 ist ferner so ausgebildet, dass sich aufgrund des Verfahrens der ersten Umlenkeinheit 76 und/oder der zweiten Umlenkeinheit 78 eine sich ergebende Distanzänderung des Lichtpfads zwischen dem jeweiligen Bildschirm 42 und dem jeweiligen Augenhintergrund 59 bei der Bestimmung der Refraktion bzw. des Nah- und/oder Fernpunkts des jeweiligen Auges ausgeglichen wird. Eine Änderung der Distanz zwischen dem Augenhintergrund 59 und dem Bildschirm 42 verfälscht das Ergebnis der Sehschärfenprüfung. Um ein verfälschtes Ergebnis zu vermeiden, wird folglich diese Distanzänderung ausgeglichen.

Der Ausgleich kann dabei auf mechanische Art und Weise so erfolgen, dass die Messeinheit 38 ebenfalls in Richtung der Y-Achse 54 unabhängig von der Ausrichteinheit 40 verfahrbar angeordnet ist. Ergibt sich eine Distanzänderung des Abstands zwischen dem Augenhintergrund 59 und dem Bildschirm 42, so wird diese Distanz durch entsprechendes Verfahren der jeweiligen Messeinheit 38 ausgeglichen. Zum Verfahren der Messeinheit 38 kann ein Verfahrantrieb vorgesehen sein, entsprechend den Antrieben, die die erste Umlenkeinheit 76 und die zweite Umlenkeinheit 78 verfahren. Sämtliche Antriebe können dabei von der Steuereinheit 64 angesteuert werden.

Ein Ausgleich kann ferner durch eine mechanische Kopplung der einzelnen verfahrbaren Bauteile so bereitgestellt werden, dass die Distanz zwischen dem Augenhintergrund 59 und dem Bildschirm 42 stets konstant ist. Andererseits ist denkbar, dass ein geregeltes System zur Verfügung gestellt wird, und zwar derart, dass je nach Verfahren der ersten Umlenkeinheit 76 und/oder der zweiten Umlenkeinheit 78 die Messeinheit 38 entsprechend nachverfahren wird, so dass die Distanz zwischen dem Augenhintergrund 59 und dem Bildschirm 42 stets konstant bleibt.

Eine weitere Art des Ausgleichs einer Distanzänderung bei der Sehschärfenmessung kann dadurch erfolgen, dass bei der Berechnung der Sehschärfe die Distanzänderung berücksichtigt wird. Dieses Verfahren beruht folglich auf einer Korrekturberechnung der Sehschärfe.

Eine weitere Möglichkeit eines Ausgleichs einer sich ergebenden Distanzänderung besteht darin, dass eine zusätzliche Linsenausgleichsanordnung vorgesehen ist, die die aufgetretene Distanzänderung im Strahlengang ausgleicht.

Dabei ist vorteilhaft, wenn eine in den Figuren nicht dargestellte Messeinrichtung zur Bestimmung der Länge des Lichtpfades zwischen dem jeweiligen Bildschirm 42 und dem jeweiligen, zugehörigen Augenhintergrund 59 vorgesehen ist. Damit kann stets die Distanz zwischen dem jeweiligen Bildschirm und dem jeweiligen Augenhintergrund 59 überprüft werden. Bei Abweichung einer vorgegebenen Soll-Distanz können zum Ausgleich entsprechende Maßnahmen getroffen werden; es kann, wie bereits ausgeführt, der Abstand verändert werden, eine Korrekturrechnung erfolgen und/oder eine geeignete Linsenanordnung verstellt werden.

Ferner wird aus insbesondere der Figur 3 deutlich, dass die zweite Umlenkeinheit 78 jeweils einen Strahlumlenker 90 aufweist, der den vom ersten Umlenkelement 76 kommenden Strahl hin zur Einblicköffnung 28 umlenkt. Im Bereich der Einblicköffnungen 28 sehen die beiden zweiten Umlenkeinheiten 78 jeweils einen Prismenkompensator 92 vor. Im Idealfall ist die Pupillenachse 94 deckungsgleich mit der Einblickachse 56. Mit dem jeweiligen Prismenkompensator 92 kann ein Winkelfehler (Phorie) zwischen einer Pupillenachse 96, wie sie in Figur 3 angedeutet ist, ausgeglichen werden. Die Prismenkompensatoren 92 dienen ferner zur Korrektur der Konvergenz beim Sehen in die Nähe.

Die jeweilige Sehschärfenlinsenanordnung 44 ist, wie in Figur 3 angedeutet, so ausgebildet, dass automatisch unterschiedliche Linsen 97 in den Strahlenpfad 50 eingebracht werden können. Dies hat den Vorteil, dass unterschiedliche Messmethoden bereitgestellt werden können, beispielsweise zur Messung mit Kreuzzylindern, einer Stabsichtigkeit (Astigmatismus), eines Zylinders sowie dessen Lage. Der Refraktionsablauf und das Einbringen der Linsen 97 kann insbesondere von der Steuereinheit 64 gesteuert und entsprechend einem vorgegebenen Meßablauf erfolgen.

Die Steuereinheit 64 ist ferner so ausgebildet, dass sie die beiden Bildschirme 42 derart ansteuert, dass die Person ein monokular dargebotenes Bild rechts oder links, dreidimensionale Bilder und/oder auch bewegte Bilder wahrnimmt. Dadurch kann eine geeignete Messung unter möglichst realistischen Bedingungen, insbesondere der Sehschärfe, erfolgen.

Zur Kommunikation mit der Person umfasst das Einblickgerät eine Kommunikationseinheit 98, die mit der Steuereinheit 64 verbunden ist. Über die Kommunikationseinheit kann die Person mit einer dritten, ortsfernen Person kommunizieren. Die Kommunikationseinheit weist dabei insbesondere eine Sprachausgabe, ggf. einen Bildschirm, sowie eine Spracherkennung auf, so dass letztlich der Person Fragen gestellt werden können, und dass die Person diese Fragen beantworten kann, wobei die Antworten weiter kommuniziert werden. Hierdurch kann eine subjektive Refraktion durchgeführt werden.

Ferner ist denkbar, dass die Kommunikationseinheit derart eingerichtet und programmiert ist, dass auch eine subjektive Refraktion ohne fachlich ausgebildetes Personal ermöglicht wird. Sämtliche wesentlichen Fragen können von der Kommunikationseinheit gestellt werden. Die Antworten, die beispielsweise über einen Auswahlkatalog vorgegeben werden können, können von der Steuereinheit 64 selbstständig ausgewertet und in logische Refraktionsschritte umgesetzt werden.

Wie insbesondere aus Figur 3 deutlich wird, ist für jedes Auge 48 ein entsprechender Lichtpfad vorgesehen, wobei die einzelnen Einheiten im Gerät für jedes Auge 48 symmetrisch zur Mittelebene 22 angeordnet sind.

Ferner ist vorteilhaft, wenn lediglich eine zentrale Steuereinheit 64 vorgesehen ist, die sämtliche Messdaten auswertet, Antriebe entsprechend ansteuert und ggf. die Kommunikation mit der Person steuert. Die Steuereinheit 64 ist dabei so eingerichtet, dass programmierte Messprogramme und insbesondere Refraktionsabläufe auf ihr ablaufen. Denkbar ist auch, dass mehrere Steuereinheiten 64 vorgesehen sind, und dass ggf. eine zentrale, übergeordnete Steuereinheit zur Koordination anderer Steuereinheiten vorgesehen ist.

Mit dem Einblickgerät 10 kann letztlich eine kompakte Baugruppe bereitgestellt werden, welche selbstständig die beiden Augen 48 einer Person vorzugsweise vollständig automatisch prüft.

## Patentansprüche

1. Binokulares Einblickgerät (10) zum insbesondere objektiven und subjektiven Prüfen der Augen (48) einer Person, mit einem Gehäuse (12), mit zwei Einblicköffnungen (28) zum Einblicken in das Gehäuse entlang jeweils einer Einblickachse (56), wobei im Gehäuse (12) für jedes Auge (48) eine Messeinheit (38) mit einem Bildschirm (42) zur Erzeugung von Sehzeichen vorgesehen ist, wobei die Messeinheit (38) für jedes Auge (48) eine verstellbare Sehschärfelinsenanordnung (44) zur Bestimmung der Refraktion des jeweiligen Auges (48) umfasst,
wobei im Gehäuse (12) für jedes Auge (48) eine entlang einer X-Achse (52) und/oder einer Y-Achse (54) verfahrbare Ausrichteinheit (40) zum Ausrichten der Einblickachse (46) hin in das jeweilige Auge (48) vorgesehen ist,
dass das Einblickgerät (10) so eingerichtet ist, dass eine durch ein Verfahren der jeweiligen Ausrichteinheit (40) sich ergebenden Distanzänderung des Lichtpfads zwischen dem Bildschirm (42) und dem Augenhintergrund (59) ausgeglichen wird, **dadurch gekennzeichnet**
**dass** eine Messeinrichtung zur Bestimmung der Länge des Lichtpfades zwischen dem Bildschirm (42) und dem Augenhintergrund (59) vorgesehen ist.

2. Einblickgerät (10) nach Anspruch 1, **dadurch gekennzeichnet, dass** die jeweilige Messeinheit (38) ferner umfasst
- eine IR-Lichtquelle (58) zur Erzeugung von definierten Lichtfiguren auf dem Augenhintergrund (59),
- einen IR-Sensor (62) zur Detektion von den am Augenhintergrund (59) reflektierten Lichtfiguren,
- einen Linsenantrieb zum Verstellen der Sehschärfelinsenanordnung (44),
und dass eine Steuereinrichtung (64) derart vorgesehen und eingerichtet ist, dass sie in Abhängigkeit von den mit dem jeweiligen IR-Sensor (62) detektierten Lichtfiguren den jeweiligen Linsenantrieb zur objektiven Bestimmung des Nah- und/oder Fernpunkts des jeweiligen Auges (48) ansteuert.

3. Einblickgerät (10) nach Anspruch 1 oder 2, **dadurch gekennzeichnet, dass** in der Messeinheit (38) ferner eine HH-Kamera (72) zur Erfassung von an der Hornhaut (73) reflektiertem Licht vorgesehen ist,
und dass wenigstens ein Ausrichtantrieb zum Verfahren der der jeweiligen Ausrichteinheit (40) vorgesehen ist,
wobei die Steuereinrichtung (64) weiter derart eingerichtet ist, dass sie in Abhängigkeit von dem von den HH-Sensoren der HH-Kamera (72) erfassten Lichts den Ausrichtantrieb derart ansteuert, dass das vom jeweiligen Bildschirm (42) erzeugte Licht entlang der Einblickachse (56) in das jeweilige Auge (48) fällt.

4. Einblickgerät (10) nach einem der vorhergehenden Ansprüche, **dadurch gekennzeichnet, dass** die Ausrichteinheit (40) eine erste Umlenkeinheit (76) und eine zweite Umlenkeinheit (78) umfasst, wobei die erste Umlenkeinheit (76) das Bildschirmlicht (42) hin zur zweiten Umlenkeinheit umleitet und die zweite Umlenkeinheit (78) das von der ersten Umlenkeinheit (76) kommende Bildschirmlicht (42) hin zur Eintrittsöffnung (28) und in das jeweilige Auge (48) umleitet, wobei die zweite Umlenkeinheit (78) quer zur Einblickachse entlang der X-Achse (52) verfahrbar angeordnet ist und wobei die erste Umlenkeinheit (76) entlang einer senkrecht zur X-Achse (72) und senkrecht zur Einblickachse verlaufenden Y-Achse (24) verfahrbar angeordnet ist.

5. Einblickgerät (10) nach Anspruch 4, **dadurch gekennzeichnet, dass** die zweite Umlenkeinheit (78) an der ersten Umlenkeinheit (76) entlang der X-Achse (52) verfahrbar angeordnet ist.

6. Einblickgerät (10) nach Anspruch 4 oder 5, **dadurch gekennzeichnet, dass** die erste Umlenkeinheit (76) an der Messeinheit (38) entlang der Y-Achse (54) verfahrbar angeordnet ist.

7. Einblickgerät (10) nach Anspruch 4, 5 oder 6, **dadurch gekennzeichnet, dass** die Messeinheiten (38) entlang der Y-Achse (54) unabhängig von der Ausrichteinheit (40) bzw. deren Umlenkeinheiten in Richtung der Y-Achse (54) verfahrbar angeordnet ist.

8. Einblickgerät (10) nach einem der Ansprüche 4 bis 7, **dadurch gekennzeichnet, dass** die Messeinheiten (38) derart eingerichtet sind, dass bei einer durch ein Verfahren der jeweiligen Umlenkeinheit (76,78) entlang der X-Achse und/oder Y-Achse sich ergebenden Distanzänderung des Lichtpfads zwischen dem jeweiligen Bildschirm (42) und dem jeweiligen Augenhintergrund (59) diese Distanzänderung durch entsprechendes Verfahren der jeweiligen Messeinheit (38) entlang der Y-Achse (54) ausgeglichen wird.

9. Einblickgerät (10) nach einem der Ansprüche 4 bis 8, **dadurch gekennzeichnet, dass** die erste Umlenkeinheit (76) einen Strahlvereiniger (82) aufweist, der das vom Bildschirm (42) kommende Bildschirmlicht und das von der IR-Lichtquelle (58) kommende IR-Licht in einem zum jeweiligen Auge (48) führenden Lichtpfad (84) vereint.

10. Einblickgerät (10) nach Anspruch 9, **dadurch gekennzeichnet, dass** der Strahlvereiniger (82) zugleich als Strahlteiler ausgebildet ist, der das an dem Augenhintergrund (59) reflektierte IR-Licht und das an der Hornhaut (73) reflektierte Licht in einen zur HH-Kamera (72) führenden Lichtpfad und einen zum IR-Sensor (62) führenden Lichtpfad teilt.

11. Einblickgerät (10) nach einem der Ansprüche 3 bis 10, **dadurch gekennzeichnet, dass** in der Messeinheit (38) ein Strahlumlenker (74) vorgesehen ist, der das an der Hornhaut (73) reflektierte Licht hin zur HH-Kamera (72) umlenkt.

12. Einblickgerät (10) nach einem der Ansprüche 3 bis 11, **dadurch gekennzeichnet, dass** im Lichtpfad des Lichts der IR-Lichtquelle (58) ein Strahlumlenker (70) vorgesehen ist, der das an dem Augenhintergrund (59) reflektierte IR-Licht aus dem Strahlenpfad (60) hin zum IR-Sensor (62) umlenkt.

13. Einblickgerät (10) nach einem der vorhergehenden Ansprüche, **dadurch gekennzeichnet, dass** eine Kommunikationseinheit (98) vorgesehen ist, die derart eingerichtet ist,
- dass Kommunikationseinheit (98) mit der Steuereinheit (64) derart kommuniziert, dass die Person über die Kommunikationseinheit mit einer dritten, ortsfernen Person kommunizieren kann oder
- dass die Person direkt mit der Steuereinheit (64) kommuniziert kann.

14. Einblickgerät (10) nach einem der vorhergehenden Ansprüche, **dadurch gekennzeichnet, dass** ein Standfuß (14) vorgesehen ist und dass das Gehäuse (12) gegenüber dem Standfuß (14) um eine Horizontalachse (16) verschwenkbar und/oder entlang einer Vertikalachse verstellbar angeordnet ist.

15. Verfahren zum direkten, entfernten und automatisierten Betreiben eines binokularen Einblickgeräts (10) nach einem der vorhergehenden Ansprüche,
**dadurch gekennzeichnet,**
**dass** die Länge des Lichtpfades zwischen dem Bildschirm (42) und dem Augenhintergrund (59) bestimmt wird, und dass eine sich durch ein Verfahren der jeweiligen Ausrichteinheit (40) Längenänderung der Länge des bestimmten Lichtpfads ausgeglichen wird.

## Claims

1. Binocular viewing device (10), in particular for the objective and subjective inspection of the eyes (48) of an individual, with a casing (12), with two viewing openings (28) for viewing into the casing along a viewing axis (56), whereby the casing (12) contains a measurement unit (38) for each eye (48) with a screen (42) for creating viewing symbols, whereby the measurement unit (38) for each eye (48) includes an adjustable vision sharpness lens attachment (44) for determining the refraction of the respective eye (48), whereby the casing (12) for each eye (48) contains an alignment unit (40) that can be moved along an X-axis (52) and/or a Y-axis (54) to align the viewing axis (46) into the respective eye (48), such that the viewing device (10) is set up such that a change in distance in the light path between the screen (42) and the ocular fundus (59) is balanced out via movement of the respective alignment unit (40), distinguished by the fact that a measurement device is provided to determine the length of the light path between the screen (42) and the ocular fundus (59).

2. Viewing device (10) as per claim 1, distinguished by the fact that the respective measurement unit (38) also includes
- an IR light source (58) for generating defined light figures on the ocular fundus (59),
- an IR sensor (62) for detecting the light figures reflected on the ocular fundus (59),
- a lens drive for adjusting the vision sharpness lens attachment (44),
and that a control unit (64) is provided and set up such that it steers the respective lens drive to the objective specification of the near and/or far point of the respective eye (48) regardless of the light figures detected with the respective IR sensor (62).

3. Viewing device (10) as per claim 1 or 2, distinguished by the fact that the measurement unit (38) also contains an HH camera (72) for recording light reflected on the cornea (73), and that at least one alignment drive for moving the respective alignment unit (40) is provided, whereby the control unit (64) is also set up such that it steers the alignment drive regardless of the light recorded by the HH sensors of the HH camera (72) such that the light generated by the respective screen (42) falls along the viewing axis (56) into the respective eye (48) .

4. Viewing device (10) as per one of the preceding claims, distinguished by the fact that the alignment unit (40) includes a first pivot unit (76) and second pivot unit (78), whereby the first pivot unit (76) redirects the screen light (42) toward the second pivot unit and the second pivot unit (78) redirects the screen light (42) coming from the first pivot unit (76) toward the input opening (28) and into the respective eye (48), whereby the second pivot unit (78) is movably positioned transversely to the viewing axis along the X-axis (52) and whereby the first pivot unit (76) is movably positioned along a Y-axis (24) perpendicularly to the X-axis (72) and perpendicularly to the viewing axis.

5. Viewing device (10) as per claim 4, distinguished by the fact that the second pivot unit (78) is movably positioned against the first pivot unit (76) along the X-axis (52).

6. Viewing device (10) as per claim 4 or 5, distinguished by the fact that the first pivot unit (76) is movably positioned against the measurement unit (38) along the Y-axis (54).

7. Viewing device (10) as per claim 4, 5 or 6, distinguished by the fact that the measurement unit (38) is movably positioned along the Y-axis (54) regardless of the alignment unit (40) or its pivot units in the direction of the Y axis (54).

8. Viewing device (10) as per one of the claims 4 to 7, distinguished by the fact that the measurement units (38) are set up such that, in the event of a distance change of the light path between the respective screen (42) and the respective ocular fundus (59) movement of the respective pivot unit (76, 78) along the X-axis and/or Y-axis, this distance change is balanced out by corresponding movement of the respective measurement unit (38) along the Y-axis (54).

9. Viewing device (10) as per one of the claims 4 to 8, distinguished by the fact that the first pivot unit (76) has a beam combiner (82) that combines the screen light coming from the screen (42) and the IR light coming from the IR light source (58) in one light path (84) leading toward the respective eye (48).

10. Viewing device (10) as per claim 9, distinguished by the fact that the beam combiner (82) is simultaneously designed as a beam diffuser that distributes the IR light reflected on the ocular fundus (59) and the light reflected on the cornea (73) in one light path leading toward the HH camera (72) and one light path leading toward the IR sensor (62).

11. Viewing device (10) as per one of the claims 3 to 10, distinguished by the fact that the measurement unit (38) contains a beam redirector (74) that redirects the light reflected on the cornea (73) toward the HH camera (72).

12. Viewing device (10) as per one of the claims 3 to 11, distinguished by the fact that the a beam redirector (70) is located in the light path of the light from the IR light source (50), that redirects the IR light reflected on the ocular fundus (59) from the beam path (60) toward the IR sensor (62).

13. Viewing device (10) as per one of the preceding claims, distinguished by the fact that a communication device (98) is provided which is set up such that
- the communication device (98) communicates with the control unit (64) such that the individual can communicate via the communication device with a third party located in a distant location, or
- the individual can communicate directly with the control unit (64).

14. Viewing device (10) as per one of the preceding claims, distinguished by the fact that a foot (14) is provided and that the casing (12) is located across from the foot (14) such that it can pivot around a horizontal axis (16) and/or along a vertical axis such that it can be adjusted.

15. Process for the direct, remote and automated operation of a binocular viewing device (10) as per one of the preceding claims, distinguished by the fact that the length of the light path between the screen (42) and the ocular fundus (59) is defined, and that a change in the length of the defined light path caused by movement of the respective alignment unit (40) is balanced out.

## Revendications

1. Testeur de vue (10) binoculaire pour examiner en particulier de manière objective et subjective les yeux (48) d'une personne, avec un boîtier (12), avec deux ouvertures de visionnage (28) pour regarder dans le boîtier le long de respectivement un axe de visionnage (56), dans lequel une unité de mesure (38) avec un écran (42) pour produire des optotypes est prévue dans le boîtier (12) pour chaque œil (48), dans lequel l'unité de mesure (38) pour chaque œil (48) comprend un dispositif d'acuité visuelle (44) réglable pour déterminer la réfraction de l'œil (48) respectif,
dans lequel une unité d'alignement (40) déplaçable le long d'un axe X (52) et/ou d'un axe Y (54) pour aligner l'axe de visionnage (46) dans l'œil (48) respectif est prévue dans le boîtier (12) pour chaque œil (48),
que le testeur de vue (10) est conçu de sorte qu'un changement de distance du trajet lumineux entre l'écran (42) et le fond d'œil (59) se produisant du fait d'un déplacement de l'unité d'alignement (40) respective est compensé, **caractérisé en ce qu'**un dispositif de mesure pour déterminer la longueur du trajet lumineux entre l'écran (42) et le fond d'œil (59) est prévu.

2. Testeur de vue (10) selon la revendication 1, **caractérisé en ce que** l'unité de mesure (38) respective comprend en outre
- une source de lumière infrarouge (58) pour générer des figures de lumière définies sur le fond d'œil (59),
- un capteur infrarouge (62) pour détecter des figures de lumière réfléchies sur le fond d'œil (59),
- un entraînement de lentille pour régler le dispositif d'acuité visuelle (44),
et qu'un dispositif de commande (64) est prévu et conçu de telle sorte que, en fonction des figures de lumière détectées avec le capteur infrarouge (62) respectif, il commande l'entraînement de lentille respectif pour déterminer de manière objective le punctum proximum et/ou remotum de l'œil (48) respectif.

3. Testeur de vue (10) selon la revendication 1 ou 2, **caractérisé en ce qu'**une caméra HH (72) pour détecter la lumière réfléchie sur la cornée (73) est prévue en outre dans l'unité de mesure (38),
et qu'au moins un entraînement d'alignement pour déplacer l'unité l'unité d'alignement (40) respective est prévu,
dans lequel le dispositif de commande (64) est conçu en outre de telle sorte que, en fonction de la lumière détectée par les capteurs HH de la caméra HH (72), il commande l'entraînement d'alignement de telle sorte que la lumière générée par l'écran (42) respectif le long de l'axe de visionnage (56) rentre dans l'œil (48) respectif.

4. Testeur de vue (10) selon l'une quelconque des revendications précédentes, **caractérisé en ce que** l'unité d'alignement (40) comprend une première unité de déviation (76) et une deuxième unité de déviation (78), dans lequel la première unité de déviation (76) dévie la lumière d'écran (42) en direction de la deuxième unité de déviation et la deuxième unité de déviation (78) dévie la lumière d'écran (42) provenant de la première unité de déviation (76) en direction de l'orifice d'entrée (28) et dans l'œil (48) respectif, dans lequel la deuxième unité de déviation (78) est disposée de manière à pouvoir se déplacer transversalement à l'axe de visionnage le long de l'axe X (52) et dans lequel la première unité de déviation (76) est disposée de manière à pouvoir se déplacer le long d'un axe Y (24) s'étendant perpendiculairement à l'axe X (72) et perpendiculairement à l'axe de visionnage.

5. Testeur de vue (10) selon la revendication 4, **caractérisé en ce que** la deuxième unité de déviation (78) est disposée de manière à pouvoir se déplacer sur la première unité de déviation (76) le long de l'axe X (52) .

6. Testeur de vue (10) selon la revendication 4 ou 5, **caractérisé en ce que** la première unité de déviation (76) est disposée sur l'unité de mesure (38) de manière à pouvoir se déplacer le long de l'axe Y (54) .

7. Testeur de vue (10) selon la revendication 4, 5 ou 6, **caractérisé en ce que** les unités de mesure (38) est disposée de manière à pouvoir se déplacer le long de l'axe Y (54) indépendamment de l'unité d'alignement (40) ou des unités de déviation de celle-ci en direction de l'axe Y (54).

8. Testeur de vue (10) selon l'une quelconque des revendications 4 à 7, **caractérisé en ce que** les unités de mesure (38) sont conçues de telle sorte que, lors d'un changement de distance du trajet lumineux entre l'écran (42) respectif et le fond d'œil (59) respectif se produisant du fait d'un déplacement de l'unité de déviation (76, 78) respective le long de l'axe X et/ou axe Y, ce changement de distance est compensé par le déplacement correspondant de l'unité de mesure (38) respective le long de l'axe Y (54).

9. Testeur de vue (10) selon l'une quelconque des revendications 4 à 8, **caractérisé en ce que** la première unité de déviation (76) présente un combineur de faisceaux (82), qui combine la lumière d'écran provenant de l'écran (42) et la lumière infrarouge provenant de la source de lumière infrarouge (58) en un trajet lumineux (84) menant à l'œil (48) respectif.

10. Testeur de vue (10) selon la revendication 9, **caractérisé en ce que** le combineur de faisceaux (82) est réalisé en même temps sous la forme d'un séparateur de faisceau, qui sépare la lumière infrarouge réfléchie sur le fond d'œil (59) et la lumière réfléchie sur la cornée (73) en un trajet lumineux menant à la caméra HH (72) et un trajet lumineux menant au capteur infrarouge (62) .

11. Testeur de vue (10) selon l'une quelconque des revendications 3 à 10, **caractérisé en ce qu'**un déflecteur de faisceau (74), qui défléchit la lumière réfléchie sur la cornée (73) en direction de la caméra HH (72), est prévu dans l'unité de mesure (38).

12. Testeur de vue (10) selon l'une quelconque des revendications 3 à 11, **caractérisé en ce qu'**un déflecteur de faisceau (70), qui défléchit la lumière infrarouge réfléchie sur le fond d'œil (59) à partir du trajet de faisceau (60) en direction du capteur infrarouge (62), est prévu dans le trajet lumineux de la lumière de la source de lumière infrarouge (58).

13. Testeur de vue (10) selon l'une quelconque des revendications précédentes, **caractérisé en ce qu'**une unité de communication (98) est prévue, qui est conçue de telle sorte
- que l'unité de communication (98) communique avec l'unité de commande (64), de telle sorte que la personne peut communiquer avec une troisième personne éloignée par l'intermédiaire de l'unité de communication ou
- que la personne peut communiquer directement avec l'unité de commande (64).

14. Testeur de vue (10) selon l'une quelconque des revendications précédentes, **caractérisé en ce qu'**un pied (14) est prévu et que le boîtier (12) est disposé de manière à pouvoir pivoter autour d'un axe horizontal (16) et/ou à pouvoir se déplacer le long d'un axe vertical par rapport au pied (14).

15. Procédé permettant le fonctionnement direct, distant et automatisé d'un testeur de vue (10) binoculaire selon l'une quelconque des revendications précédentes,
**caractérisé en ce**
**que** la longueur du trajet lumineux entre l'écran (42) et le fond d'œil (59) est déterminée, et qu'un changement de longueur de la longueur du trajet lumineux déterminé, du fait d'un déplacement de l'unité d'alignement (40) respective, est compensé.
